(19) **Europäisches Patentamt / European Patent Office / Office européen des brevets**

(11) **EP 3 187 479 A1**

(12) # EUROPEAN PATENT APPLICATION
published in accordance with Art. 153(4) EPC

(43) Date of publication:
**05.07.2017 Bulletin 2017/27**

(21) Application number: **15836807.6**

(22) Date of filing: **07.08.2015**

(51) Int Cl.:
$C07C\ 27/16^{(2006.01)}$    $C07C\ 29/48^{(2006.01)}$
$C07C\ 31/125^{(2006.01)}$    $C07C\ 45/40^{(2006.01)}$
$C07C\ 49/04^{(2006.01)}$    $C07C\ 51/34^{(2006.01)}$
$C07C\ 53/126^{(2006.01)}$    $B01F\ 3/04^{(2006.01)}$
$B01F\ 13/00^{(2006.01)}$    $B01J\ 32/00^{(2006.01)}$
$C07B\ 61/00^{(2006.01)}$

(86) International application number:
**PCT/JP2015/072503**

(87) International publication number:
**WO 2016/031526 (03.03.2016 Gazette 2016/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **29.08.2014 JP 2014175313**

(71) Applicants:
• **Osaka Prefecture University Public Corporation**
  **Sakai-shi, Osaka 599-8231 (JP)**
• **Daicel Corporation**
  **Osaka-shi, Osaka 530-0011 (JP)**

(72) Inventors:
• **MUTO, Akinori**
  **Sakai-shi**
  **Osaka 599-8531 (JP)**
• **MURAI, Yoshiyuki**
  **Himeji-shi**
  **Hyogo 671-1283 (JP)**
• **SUZUKI, Takamasa**
  **Himeji-shi**
  **Hyogo 671-1283 (JP)**
• **TAKASE, Ichiro**
  **Himeji-shi**
  **Hyogo 671-1283 (JP)**

(74) Representative: **Grünecker Patent- und Rechtsanwälte PartG mbB Leopoldstraße 4 80802 München (DE)**

(54) **OXIDATION REACTOR AND PROCESS FOR PRODUCING OXIDE**

(57) Provided is an oxidation reactor capable of oxidizing hydrocarbons with both reaction efficiency and energy efficiency. The oxidation reactor according to the present invention includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. In the flow reactor, an oxidation catalyst is immobilized or packed. The gas-liquid mixing unit houses, in its channel, a mobile particle which is capable of rotating and/or moving to mix the liquid with the gas to thereby form a gas-liquid slug flow. The gas-liquid slug flow is introduced into the flow reactor.

[Fig. 1]

EP 3 187 479 A1

# Description

## Technical Field

The present invention relates to oxidation reactors and methods for producing oxides using the oxidation reactors. More specifically, the present invention relates to an oxidation reactor for use in oxidation of a hydrocarbon, which is an organic substrate, in the presence of oxygen and ozone to give an oxide; and to a method for producing an oxide using the oxidation reactor. This application claims priority to Japanese Patent Application No. 2014-175313, filed August 29, 2014 to Japan, the entire contents of which are incorporated herein by reference.

## Background Art

Oxidation reactions are one of most basic reactions in the organic chemical industry, for which a variety of oxidation methods have been developed. Patent Literature (PTL) 1 describes an oxidation method by which a compound capable of forming a radical is oxidized typically with molecular oxygen by the catalysis of a lipid-soluble imide compound. With this method, a wide variety of organic substrates can be oxidized to give corresponding oxides. Disadvantageously, however, this method offers a low yield when hydrocarbons such as straight chain alkanes are used as the substrate.

An oxidation reaction of a hydrocarbon with oxygen is generally performed by introducing the hydrocarbon as a starting material into a stirred-tank reactor, and blowing oxygen or air through nozzles into the reactor. This method, however, offers poor heat transfer and low gas-liquid mixing efficiency and is not always satisfactory in reaction efficiency and energy efficiency.

## Citation List

## Patent Literature

PTL 1: Japanese Unexamined Patent Application Publication (JP-A) No. 2002-331242

## Summary of Invention

## Technical Problem

Accordingly, the present invention has an object to provide an oxidation reactor that is capable of oxidizing hydrocarbons with good reaction efficiency and good energy efficiency.

The present invention has another object to provide a method for efficiently producing a corresponding oxide or oxides from a hydrocarbon.

## Solution to Problem

After intensive investigations to achieve the objects, the inventors of the present invention have found that a specific oxidation reactor enables oxidation of a hydrocarbon with good reaction efficiency and good energy efficiency. This oxidation reactor includes a liquid inlet channel to introduce the hydrocarbon, a gas inlet channel to introduce oxygen and ozone, a gas-liquid mixing unit to mix the liquid with the gases, and a flow reactor in which an oxidation catalyst is immobilized or packed. The gas-liquid mixing unit houses, in its channel, a mobile particle to mix the liquid with the gases to thereby form a gas-liquid slug flow. The present invention has been made based on these findings.

Specifically, the present invention relates to followings.

(1) The present invention relates to an oxidation reactor that includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. The flow reactor bears an oxidation catalyst that is immobilized to or packed in the flow reactor. The gas-liquid mixing unit houses, in its channel, a mobile particle capable of rotating and/or moving to mix the liquid with the gas to thereby form a gas-liquid slug flow. The gas-liquid slug flow is introduced into the flow reactor.

(2) The oxidation reactor according to (1) may further include a gas-liquid separator downstream from the flow reactor.

(3) The oxidation reactor according to (2) may further include a circulation channel, through which at least part of a liquid separated in the gas-liquid separator is recycled to the gas-liquid mixing unit or to an upstream portion from the gas-liquid separator.

(4) The oxidation reactor according to one of (2) and (3) may further include a circulation channel, through which at least part of a gas separated in the gas-liquid separator is recycled to the gas-liquid mixing unit or to an upstream portion from the gas-liquid mixing unit.

(5) In the oxidation reactor according to any one of (1) to (4), the oxidation catalyst may be a catalyst including a transition metal in the form of an elementary substance, a compound, or an ion; and an inorganic support onto which the transition metal is supported or immobilized.

(6) In the oxidation reactor according to any one of (1) to (4), the oxidation catalyst may be selected from a catalyst including a transition metal compound, and a support having a Hammett acidity function ($H_0$) of

-9 or less and supporting the transition metal compound; and a catalyst including a transition metal ion, and a support having a Hammett acidity function ($H_0$) of -9 or less and being ion-exchanged with the transition metal ion.

(7) In the oxidation reactor according to any one of (1) to (6), the mobile particle may be made of a material selected from glass, resins, and metals.

(8) In the oxidation reactor according to any one of (1) to (7), the mobile particle may be approximately spherical in shape.

(9) In the oxidation reactor according to (8), the mobile particle may have a diameter of 0.3 to 1.5 mm.

(10) In the oxidation reactor according to any one of (5) to (9), the transition metal compound may be a compound containing, as the transition metal, at least one transition metal selected from the group consisting of Co, Mn, Fe, Zr, Ce, V, and Mo; or the transition metal ion may be at least one ion selected from the group consisting of cobalt ions, manganese ions, vanadium ions, iron ions, zirconium ions, and cerium ions.

(11) In the oxidation reactor according to any one of (5) to (10), the transition metal compound may be selected from cobalt compounds and manganese compounds; or the transition metal ion may be selected from cobalt ions and manganese ions.

(12) In the oxidation reactor according to any one of (6) to (11), the support may include a strongly acidic or super acidic ion exchange resin.

(13) In the oxidation reactor according to any one of (6) to (12), the support may include a fluorinated sulfonic acid resin.

(14) In the oxidation reactor according to any one of (1) to (4), and (6) to (13), the flow reactor may have a coating on an inner wall of the flow reactor, where the coating includes a strongly acidic or super acidic ion exchange resin being ion-exchanged with a transition metal ion.

(15) The oxidation reactor according to any one of (1) to (14) may further include an imide compound inlet channel upstream from the flow reactor, where an imide compound having a cyclic imide skeleton is introduced from the imide compound inlet channel.

(16) In the oxidation reactor according to any one of (1) to (15), the hydrocarbon is at least one selected from the group consisting of aliphatic hydrocarbons, alicyclic hydrocarbons, and aromatic hydrocarbons.

(17) The present invention also relates to a method for producing an oxide. The method includes oxidizing a hydrocarbon in the coexistence of oxygen and ozone using the oxidation reactor according to any one of (1) to (16), to yield a corresponding oxide.

Advantageous Effects of Invention

According to the present invention, a mobile particle is disposed in a channel (gas-liquid mixing zone) of the gas-liquid mixing unit and rotates and/or moves to form a fine gas-liquid slug flow (or gas-liquid alternate flow), and this gas-liquid slug flow is introduced into the flow reactor bearing or including the oxidation catalyst. Further, both oxygen and ozone are used as oxidizers. Thus, reaction components can move quickly, and the oxidation can proceed rapidly at a high rate. Accordingly, with the present invention, a wide variety of hydrocarbons, in particular even straight chain alkanes, can be efficiently oxidized. Further, the reaction heat can be efficiently removed, and high energy efficiency is obtained, because of smooth heat exchange. In addition and advantageously, the oxidation apparatus can be downsized.

Brief Description of Drawings

Fig. 1 is a schematic flow diagram illustrating an oxidation reactor according to an embodiment of the present invention;
Fig. 2 is a schematic view of a gas-liquid mixing unit when viewed laterally, where the upside of the figure corresponds to the upside of the gas-liquid mixing unit;
Fig. 3 is a schematic view of the gas-liquid mixing unit when viewed from above;
Fig. 4 is a schematic diagram illustrating a gas-liquid slug flow (gas-liquid alternate flow) formed in a model experiment of Example 1; and
Fig. 5 is a schematic diagram illustrating a gas-liquid slug flow (gas-liquid alternate flow) formed in a model experiment of Comparative Example 1.

Description of Embodiments

Oxidation Reactor

The oxidation reactor according to the present invention includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. The flow reactor houses or bears an oxidation catalyst that is immobilized to or packed in the flow reactor. The oxidation reactor further includes a mobile particle disposed in a channel of the gas-liquid mixing unit. The mobile particle is capable of rotating and/or moving to mix the liquid with the gas to thereby form a gas-liquid slug flow (or gas-liquid alternate flow). The gas-liquid slug flow is introduced into the flow reactor, in which the hydrocarbon is oxidized in the coexistence of oxygen and ozone.

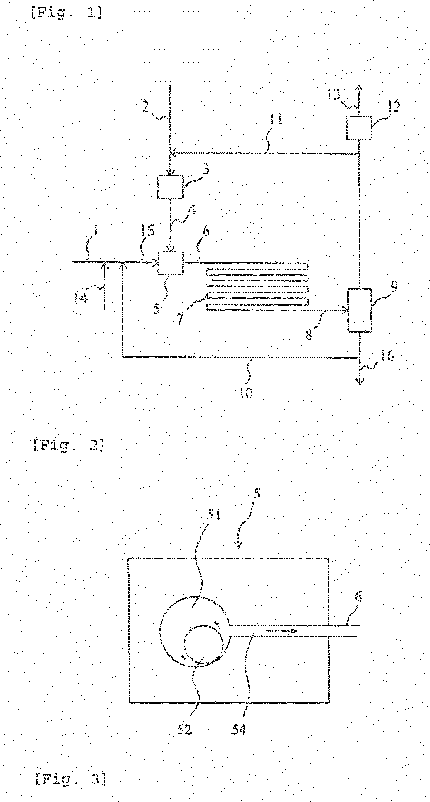

Fig. 1 is a schematic flow diagram illustrating an oxidation reactor according to an embodiment of the present invention. Fig. 2 is an exemplary illustration of a gas-liquid mixing unit 5 in Fig. 1 and is a schematic view of the gas-liquid mixing unit 5 when viewed laterally. The upside of Fig. 2 corresponds to the upside of the gas-liquid mixing unit 5. Fig. 3 is a schematic view of the gas-liquid mixing unit 5 illustrated in Fig. 2, when viewed from above.

In this embodiment, the reaction substrate hydrocarbon is fed through a hydrocarbon supply channel 1 to the gas-liquid mixing unit (gas-liquid mixer) 5. Where necessary, the hydrocarbon may be fed in the form of a solution in a solvent. On the other hand, oxygen or an oxygen-containing gas (such as air) is fed through an oxygen inlet channel 2 to an ozone generator 3 and partially converted into ozone to form a mixed gas containing oxygen and ozone, and the mixed gas is fed through a gas inlet channel 4 to the gas-liquid mixing unit 5. The mixed gas containing oxygen and ozone may be mixed with an inert gas such as nitrogen gas before being fed to the gas-liquid mixing unit 5.

The gas-liquid mixing unit 5 includes a channel (gas-liquid mixing zone) 51 in which the gas and the liquid are mixed with each other. The channel 51 houses a mobile particle 52 which is disposed rotatably and/or movably. The rotation and/or movement of the mobile particle 52 within the channel 51 mixes and disperses the liquid fed through the liquid inlet channel 15 with the gas fed through the gas inlet channel 4 to form a gas-liquid slug flow (gas-liquid alternate flow). The channel 51 is coupled to an inlet channel 53 and an outlet channel 54. Through the inlet channel 53, the gas and the liquid flow into the channel 51. Through the outlet channel 54, a gas-liquid mixture formed in the channel 51 is discharged. The gas inlet channel 4 and the liquid inlet channel 15 are coupled to the inlet channel 53. The hydrocarbon supply channel 1 is merged with a liquid circulation channel 10 and an imide compound inlet channel 14 and leads to the liquid inlet channel 15. The outlet channel 54 is coupled to a gas-liquid mixture channel 6, which leads to a flow reactor 7. The outlet channel 54 may be coupled directly to the flow reactor 7.

A material to constitute the gas-liquid mixing unit 5 is not limited, as long as being inert to and insoluble in the gas and the liquid to be used in the reaction. Non-limiting examples of the material for use herein include resins exemplified by fluorocarbon resins (such as Teflon® and Daiflon), polyester resins, polyolefin resins, polycarbonate resins, polystyrene resins, polyamide resins, and polyimide resins; glass; and metals exemplified by titanium, and alloys such as stainless steels. The gas-liquid mixing unit 5, when made typically of a metal, may bear a resin coating and/or glass lining in a portion where the unit is in contact with the gas-liquid mixture.

The channel 51 disposed in the gas-liquid mixing unit 5 may have any shape, as long as not impeding the movement of the mobile particle 52, but is generally cylindrical (round tubular) in shape. The channel 51, when being cylindrical, is preferably disposed so that the axis of the cylinder lies in the horizontal direction (is preferably disposed laterally). In this case, the fluids flow in the axial direction of the cylinder (namely, flows horizontally). The channel 51 has only to have such a size that the mobile particle 52 can smoothly rotate and/or move and the formed gas-liquid mixture can be rapidly discharged from the channel 51 and fed to the flow reactor 7. For example, the channel 51, when being cylindrical, has e an inside diameter of typically about 0.5 to about 3 mm, preferably 1 to 2.5 mm, and more preferably 1.5 to 2.3 mm and has a length of typically 1 to 6 mm, preferably 2 to 5 mm, and more preferably 2.5 to 3.5 mm. The channel 51 does not always have to have a circular cross-section. In general, the channel 51 has an equivalent circle diameter of typically 0.5 to 3 mm, preferably 1 to 2.5 mm, and more preferably 1.5 to 2.3 mm. As used herein, the term "equivalent circle diameter" refers to the diameter of a circle having an area equal to the cross-sectional area of the channel 51.

A material to constitute the mobile particle 52 is not limited, as long as being inert to and insoluble in the gas and the liquid to be used in the reaction. The material may be any one such as glass, resins, and metals, but is preferably glass. The mobile particle 52 is preferably approximately spherical or globular in shape. The mobile particle 52, when being spherical in shape, has a diameter of typically 0.3 to 1.5 mm, and preferably 0.5 to 1.4 mm. The diameter of the mobile particle 52 is preferably 0.3 to 0.9 time, and more preferably 0.4 to 0.7 time, as much as the equivalent circle diameter of the channel 51. This is preferred for efficiently mixing the gas with the liquid. The number of the mobile particle 52 may be selected within such a range as not adversely affect the formation of the gas-liquid slug flow by mixing of the gas with the liquid, and is typically 1 to 5, and preferably 1.

The inlet channel 53, the outlet channel 54, and the gas-liquid mixture channel 6 each independently have an inside diameter of typically 0.4 to 1.8 mm, and preferably 0.7 to 1.6 mm. The inlet channel 53 and the outlet channel 54 preferably have inside diameters each smaller than the diameter of the mobile particle 52 so as to prevent the mobile particle 52 from entering the inlet channel 53 and the outlet channel 54. When the channel 51 is cylindrical, the cylindrical channel is preferably coupled to the inlet channel 53 at one end in the lengthwise direction of the cylinder, and coupled to the outlet channel 54 at the other end.

To the channel 51 of the gas-liquid mixer 5, the mixed gas containing oxygen and ozone and the liquid containing the substrate hydrocarbon are fed, and are mixed with each other through gas-liquid mixing by the rotation and/or movement of the mobile particle 52 within the channel 51 to form a gas-liquid slug flow (gas-liquid alternate flow), and the gas-liquid slug flow is discharged from the outlet channel 54 and flows via the gas-liquid mixture channel 6 through the flow reactor 7. In the flow reactor 7, the substrate hydrocarbon is oxidized to form a corresponding oxide or oxides (oxidation product or products). Non-limiting examples of the oxides include alcohols, ketones, aldehydes, carboxylic acids, and hydroperoxides.

The oxidation catalyst, as described below, is immobilized to or packed in the flow reactor 7. A material to constitute the flow reactor is not limited, as long as being inert to and insoluble in the gas and the liquid for use in the reaction. Non-limiting examples of the material include resins exemplified by fluorocarbon resins such as Teflon® and Daiflon, polyester resins, polyolefin resins, polycarbonate resins, polystyrene resins, polyamide resins, and polyimide resins; glass; and metals exemplified by titanium, and alloys such as stainless steels. The flow reactor 7, when being made typically of a metal, may bear a resin coating and/or a glass lining in a portion to be in contact with the gas-liquid mixture.

The flow reactor 7 has an inside diameter of typically 0.3 to 3 mm, preferably 0.4 to 1.8 mm, and more preferably 0.7 to 1.6 mm. When the flow reactor bears a coating typically of a catalyst layer, the term "inside diameter" refers to the diameter of the inner space excluding the coating. The flow reactor 7 has a length of generally about 0.1 to about 20 m, and preferably about 0.3 to about 10 m, while the length can be selected as appropriate according typically to the reaction rate.

The temperature control of the flow reactor may be performed typically by configuring the flow reactor as having a multiple-tube structure and allowing a heat medium or coolant to pass though the inner tube or the outer tube; or by immersing the flow reactor in a heat medium bath or a coolant bath.

The reaction mixture discharged from the flow reactor 7 is introduced from a reaction mixture channel 8 into a gas-liquid separator 9 and is separated into a gas and a liquid. The flow reactor 7 may be coupled directly to the gas-liquid separator 9. The separated gas is introduced into an ozone decomposer 12 and then discharged through an exhaust gas line 13 to the outside. At least part of the separated gas may be returned, as needed, through a gas circulation channel 11 typically to a portion upstream from the ozone generator 3 and recycled to the reaction system. Meanwhile, at least part of the liquid separated in the gas-liquid separator 9 can be recycled through the liquid circulation channel 10 and the liquid inlet channel 15 to the gas-liquid mixing unit 5, typically using a pump. The liquid separated in the gas-liquid separator 9 can be recovered through a liquid recovery channel 16. Where necessary, the imide compound inlet channel 14 may be disposed upstream from the flow reactor 7, preferably in the gas-liquid mixing unit 5 or in a portion upstream from the gas-liquid mixing unit 5. Through the imide compound inlet channel 14, an imide compound having a cyclic imide skeleton is introduced.

Oxygen and Ozone

According to the present invention, oxygen, which serves as an oxidizer, is used in the coexistence of ozone. A mixed gas containing oxygen gas and ozone gas is used in the reaction. The combination use of oxygen, which serves as an oxidizer, with ozone can promote withdrawal of hydrogen from the substrate hydrocarbon and activate the radical reaction. This can accelerate the oxidation reaction even under mild conditions. From the viewpoints of reactivity and economic efficiency, the mixed gas containing oxygen gas and ozone gas may contain the ozone gas in a proportion of typically about 0.1 to about 10 volume percent relative to the oxygen gas. The oxygen and ozone may each be diluted with an inert gas such as nitrogen gas to an appropriate concentration before use. The mixed gas containing oxygen gas and ozone gas may contain the oxygen gas in a concentration of typically 5 volume percent or more, preferably 10 volume percent or more, and more preferably 15 volume percent or more. The upper limit of the concentration is typically 99.9 volume percent, preferably 80 volume percent, more preferably 50 volume percent, and particularly preferably 25 volume percent.

Reaction Substrate

The present invention uses a hydrocarbon as a reaction substrate (reactant). Non-limiting examples of the hydrocarbon include aliphatic hydrocarbons, alicyclic hydrocarbons, and aromatic hydrocarbons.

Non-limiting examples of the aliphatic hydrocarbons include straight chain alkanes such as ethane, propane, butane, pentane, hexane, heptane, octane, nonane, decane, undecane, dodecane, tridecane, tetradecane, pentadecane, hexadecane, heptadecane, octadecane, nonadecane, icosane, henicosane, docosane, triacontane, and tetracontane, of which $C_3$-$C_{30}$, preferably $C_4$-$C_{20}$, straight chain alkanes are typified; branched chain alkanes such as 2-methylpropane, 2-methylbutane, 2,2-dimethylpropane, 2-methylpentane, 3-methylpentane, 2,3-dimethylbutane, 2-methylhexane, 3-methylhexane, 3,4-dimethylhexane, and 3-methyloctane, of which $C_3$-$C_{30}$, preferably $C_4$-$C_{20}$, branched chain alkanes are typified; and straight or branched chain alkenes or alkadienes, such as propylene, isobutylene, 1-pentene, 1-hexene, 2-hexene, 1-heptene, 1-octene, 1-decene, 1-dodecene, 1-tetradecene, and 1,4-hexadiene, of which $C_3$-$C_{30}$, preferably $C_4$-$C_{20}$, straight or branched chain alkenes or alkadienes are typified.

Non-limiting examples of the alicyclic hydrocarbons include cycloalkanes such as cyclopropane, cyclobutane, cyclopentane, cyclohexane, cycloheptane, cyclooctane, cyclononane, cyclodecane, cyclododecane, cyclotetradecane, cyclohexadecane, and cyclotriacontane, of which cycloalkanes containing 3 to 30 members (preferably 5 to 30 members, and particularly preferably 5 to 20 members) are typified; cycloalkenes such as cyclopropene, cyclobutene, cyclopentene, cyclooctene, cyclohexene, cycloheptene, and cyclododecene, of which cycloalkenes containing 3 to 30 members (preferably 3 to 20 members, and particularly preferably 3 to 12 members) are typified; cycloalkadienes such as cyclopentadiene, 1,3-cyclohexadiene, and 1,5-cyclooctadiene; cycloalkatrienes such as cyclooctatriene; and bicyclic, tricyclic, or tetracyclic bridged hydrocarbons such as decalin (decahydronaphthalene), bicyclo[2.2.0]hexane, bicyclo[2.2.2]octane, bicyclo[3.2.1]octane, bicyclo[4.3.2]undecane, bicyclo[3.3.3]undecane, norbornane, norbornene, tricyclo[5.2.1.0$^{2,6}$]decane, tricyclo[6.2.1.0$^{2,7}$]undecane, adamantane, perhydroanthracene, perhydroacenaphthene, perhydrophenanthrene, perhydrophenalene, and perhydroindene.

Non-limiting examples of the aromatic hydrocarbons include $C_6$-$C_{20}$ aromatic hydrocarbons, such as benzene, naphthalene, and anthracene.

The hydrocarbon may have, as substituents, one or more groups selected from the group consisting of aliphatic hydrocarbon groups, alicyclic hydrocarbon groups, and aromatic hydrocarbon groups.

Examples of the aliphatic hydrocarbon groups include monovalent or higher-valent aliphatic hydrocarbon groups which are groups resulting from removing one or more hydrogen atoms each from the structural formulae of the aliphatic hydrocarbons. Non-limiting examples of the monovalent aliphatic hydrocarbon groups include $C_1$-$C_{10}$ straight or branched chain alkyls such as methyl, ethyl, propyl, isopropyl, butyl, isobutyl, s-butyl, t-butyl, pentyl, hexyl, octyl, and decyl; $C_2$-$C_{10}$ alkenyls such as vinyl, isopropenyl, and 1-butenyl; and $C_2$-$C_{10}$ alkynyls such as ethynyl and propynyl. Non-limiting examples of divalent aliphatic hydrocarbon groups include $C_1$-$C_{10}$ straight or branched chain alkylene groups such as methylene, ethylene, propylene, trimethylene, isopropylidene, and tetramethylene groups. Non-limiting examples of trivalent aliphatic hydrocarbon groups include $C_1$-$C_{10}$ alkanetriyls such as 1,2,3-propanetriyl.

Examples of the alicyclic hydrocarbon groups include monovalent or higher-valent alicyclic hydrocarbon groups which are groups resulting from removing one or more hydrogen atoms each from the structural formulae of the alicyclic hydrocarbons. Non-limiting examples of the alicyclic hydrocarbon groups include cycloalkyls such as cyclopentyl and cyclohexyl; cycloalkenyls such as cyclopentenyl and cyclohexenyl; and bridged hydrocarbon groups such as adamant-1-yl, norborn-2-yl, and norbornane-7,7-diyl.

Examples of the aromatic hydrocarbon groups include monovalent or higher-valent aromatic hydrocarbon groups which are groups resulting from removing one or more hydrogen atoms each from the structural formulae of the aromatic hydrocarbons. Non-limiting examples of the aromatic hydrocarbon groups include phenyl, 1-naphthyl, 1,2-phenylene, 1,3-phenylene, and 1,4-phenylene groups.

The "hydrocarbon" also includes compounds each derived from an alicyclic hydrocarbon and an aromatic hydrocarbon fused to each other in such a manner as to have two carbon atoms in common. Non-limiting examples of the compounds of this category include indane, tetrahydronaphthalene, fluorene, and acenaphthene.

The hydrocarbon may have one or more substituents other than hydrocarbon groups within ranges not adversely affecting the reaction, or not.

The hydrocarbon for use in the present invention as a reaction substrate may contain carbon atoms in any number not limited, but contains preferably about 2 to about 30 carbon atoms, more preferably 3 to 25 carbon atoms, and furthermore preferably 4 to 20 carbon atoms.

Preferred, but non-limiting examples of the hydrocarbon for use in the present invention include straight chain alkanes such as propane, butane, pentane, hexane, heptane, octane, nonane, decane, undecane, dodecane, tridecane, tetradecane, pentadecane, hexadecane, heptadecane, octadecane, nonadecane, and icosane, of which $C_3$-$C_{30}$, preferably $C_4$-$C_{20}$, straight chain alkanes are typified; branched chain alkanes such as 2-methylpropane, 2-methylbutane, 2,2-dimethylpropane, 2-methylpentane, 3-methylpentane, 2,3-dimethylbutane, 2-methylhexane, 3-methylhexane, 3,4-dimethylhexane, and 3-methyloctane, of which $C_3$-$C_{30}$, preferably $C_4$-$C_{20}$, branched chain alkanes are typified; cycloalkanes such as cyclopentane and cyclohexane, of which cycloalkanes containing 3 to 30 members (preferably 5 to 30 members, and particularly preferably 5 to 20 members) are typified; aromatic hydrocarbons each containing one or more alkyls (e.g., $C_1$-$C_6$ alkyls) bonded to an aromatic ring, such as toluene, o-xylene, m-xylene, p-xylene, o-t-butyltoluene, m-t-butyltoluene, p-t-butyltoluene, 1-ethyl-4-methylbenzene, 1-ethyl-3-methylbenzene, 1-isopropyl-4-methylbenzene, 1-t-butyl-4-methylbenzene, mesitylene, pseudocumene, durene, methylnaphthalene, dimethylnaphthalene, methylanthracene, 4,4'-dimethylbiphenyl, ethylbenzene, propylbenzene, butylbenzene, and 1,4-diethylbenzene; and compounds each including an alicyclic hydrocarbon and an aromatic hydrocarbon fused to each other in such a manner as to have two carbon atoms in common, such as tetrahydronaphthalene and fluorene.

In particular, the present invention enables efficient oxidation even of straight chain alkanes, which resist efficient oxidation by conventional techniques. Thus, the present invention is useful particularly when straight chain alkanes are used as the substrates.

The reaction substrate hydrocarbon may be diluted to an appropriate concentration with a solvent that is inert to the reaction, before use. However, the present invention allows the oxidation reaction to proceed even when approximately no solvent is used, because the oxidizer oxygen is used in the coexistence of ozone. The use of approximately no solvent eliminates or minimizes the need for separation of the reaction product oxide from the solvent and contributes to simplification of the production process.

Non-limiting examples of the solvent include organic acids such as acetic acid and propionic acid; nitriles such as acetonitrile, propionitrile, and benzonitrile; amides such as formamide, acetamide, dimethylformamide, and dimethylacetamide; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents.

The liquid to be fed to the gas-liquid mixing unit 5 (or the flow reactor 7) may contain the reaction substrate hydrocarbon in a concentration of preferably 30 weight percent or more, more preferably 50 weight percent or more, furthermore preferably 85 weight percent or more, and particularly preferably 95 weight percent or more. This is preferred from the viewpoint of reaction efficiency.

Oxidation Catalyst

The oxidation catalyst for use in the present invention is not limited, as long as offering catalysis to the oxidation reaction, but is preferably selected from catalysts each including a transition metal in the form of an elementary substance, a compound, or an ion, and an inorganic support onto which the transition metal is supported or immobilized. From the viewpoint of reaction rate, the oxidation catalyst is preferably selected from catalysts each including a transition metal compound, and a support having a Hammett acidity function ($H_0$) of -9 or less and supporting the transition metal compound; and catalysts each including a transition metal ion, and the support being ion-exchanged with the transition metal ion.

The transition metal is often selected from metal elements belonging to Groups 3 to 12 of the periodic table. Non-limiting examples of the metal elements include Group 3 elements such as Sc, lanthanoids, and actinoids; Group 4 elements such as Ti, Zr, and Hf; Group 5 elements such as V; Group 6 elements such as Cr, Mo, and W; Group 7 elements such as Mn; Group 8 elements such as Fe and Ru; Group 9 elements such as Co and Rh; Group 10 elements such as Ni, Pd, and Pt; Group 11 elements such as Cu; and Group 12 elements such as Zn, each of the periodic table. Preferred metal elements include elements belonging to Groups 5 to 11 of the periodic table and are exemplified by, but not limited to, elements belonging to Groups 5 to 9, such as Co, Mn, Fe, V, and Mo. The metal elements may have any valence not limited and has a valence of typically 0 to about 6, and preferably 2 or 3.

Examples of the transition metal compound include inorganic compounds and organic compounds of the metal elements. Non-limiting examples of the inorganic compounds include elementary substances, hydroxides, oxides (including complex oxides), halides (fluorides, chlorides, bromides, and iodides), oxoacid salts (such as nitrates, sulfates, phosphates, borates, and carbonates), salts of isopolyacids, and salts of heteropolyacids. Non-limiting examples of the organic compounds include complexes, and organic acid salts exemplified by acetates, propionates, cyanides, naphthenates, and stearate; alkylsulfonates such as methanesulfonates, ethanesulfonates, octanesulfonates, and dodecanesulfonates, of which $C_6$-$C_{18}$ alkylsulfonates are typified; and arylsulfonates which may be substituted with alkyl, such as benzenesulfonates, p-toluenesulfonates, naphthalenesulfonates, decylbenzenesulfonates, and dodecylbenzenesulfonates, of which $C_6$-$C_{18}$ alkylarylsulfonates are typified. Non-limiting examples of ligands constituting the complexes include OH (hydroxo), alkoxys (such as methoxy, ethoxy, propoxy, and butoxy), acyls (such as acetyl and propionyl), alkoxycarbonyls (such as methoxycarbonyl and ethoxycarbonyl), acetylacetonato, cyclopentadienyl, halogen atoms (such as chlorine and bromine), CO, CN, oxygen atom, $H_2O$ (aquo), phosphorus compounds exemplified by phosphines (such as triphenylphosphine and other triarylphosphines), and nitrogen-containing compounds such as $NH_3$ (ammine), NO, $NO_2$ (nitro), $NO_3$ (nitrato), ethylenediamine, diethylenetriamine, pyridine, and phenanthroline.

Specific, but non-limiting examples of the transition metal compound include, when taking cobalt compounds as examples, divalent or trivalent cobalt compounds exemplified by inorganic compounds such as cobalt hydroxides, cobalt oxides, cobalt chlorides, cobalt bromides, cobalt nitrate, cobalt sulfates, and cobalt phosphate; organic acid salts such as cobalt acetate, cobalt naphthenate, and cobalt stearate; and complexes such as acetylacetonatocobalt. Non-limiting examples of manganese compounds include di- to penta-valent manganese compounds exemplified by inorganic compounds such as manganese hydroxides, manganese oxides, manganese chlorides, and manganese sulfates; and complexes such as acetylacetonatomanganese. Examples of compounds of other transition metal elements include compounds corresponding to the cobalt or manganese compounds. The oxidation catalyst may include each of different transition metal compounds alone or in combination. The combination use of two or more transition metal compounds having different valences (such as the combination use of a divalent metal compound and a trivalent metal compound) is also preferred. For restraining deterioration in catalytic activity, the transition metal compound for use in the present invention preferably includes one or more compounds containing at least one transition metal selected from Co, Mn, Fe, Zr, Ce, V, and Mo; and more preferably includes one or more compounds selected from cobalt compounds and manganese compounds, of which organic acid salts are preferred. In particular, the support preferably includes both a cobalt compound and a manganese in combination.

The transition metal ion for use herein preferably includes one or more ions selected typically from cobalt ions, manganese ions, vanadium ions, iron ions, zirconium ions, and cerium ions, and particularly preferably includes a cobalt ion, or a manganese ion, or both (a cobalt ion in combination with a manganese ion).

In an embodiment, the catalyst includes the transition metal in the form of an elementary substance, a compound, or an ion, and an inorganic support onto which the transition metal is supported or immobilized. Non-limiting examples of the "inorganic support" in the catalyst include zeolite, silica ($SiO_2$), alumina ($Al_2O_3$), silica-alumina ($SiO_2$-$Al_2O_3$), and activated carbon.

The support having a Hammett acidity function ($H_0$) of -9 or less has only to have a Hammett acidity function ($H_0$) of -9 or less at least in its surface. Non-limiting examples of such support include sulfuric acid-supporting solids which are solids (such as $Al_2O_3$, $SiO_2$ zeolite, $SiO_2$-$Al_2O_3$, polymers, graphite, and metals) supporting concentrated sulfuric acid; solid strong acids; and solid super strong acids (acids having a Hammett acidity function ($H_0$) of less than -11.93). The concentrated sulfuric acid has a Hammett acidity function $H_0$ of -9.88 at 96%, of -10.27 at 98%, and of -11.93 at 100%.

Non-limiting examples of the solid strong acids and solid super strong acids include immobilized liquid super strong acids each containing a liquid super strong acid (such as $SbF_5$, $BF_3$, $BF$-$SbF_5$, $FSO_3H$-$SbF_5$, or $TaF_5$) supported on a solid (such as $Al_2O_3$, $SiO_2$, zeolite, $SiO_2$-$Al_2O_3$, a polymer, graphite, or a metal); binary metal salts prepared by grinding and mixing $AlCl_3$ or $AlBr_3$ with one selected from $CuSO_4$, $CuCl_2$, $Ti_2(SO_4)_3$, and $TiCl_3$; sulfated metal oxides including a sulfate ion supported by and bonded to a metal oxide (such as $Fe_2O_3$ $TiO_2$, $ZrO_2$, $HfO_2$, $SnO_2$ $Al_2O_3$, or $SiO_2$) via adsorption and firing; noble metals/sulfated metal oxides including the sulfated metal oxide mixed with a noble metal such as Ir or Pt; metal oxide super strong acids formed by allowing a metal oxide (such as $ZrO_2$ $SnO_2$, $TiO_2$, or $Fe_2O_3$) to adsorb, for example, $WO_3$, $MoO_3$, or $B_2O_3$, and firing the resulting article at a high temperature; strongly acidic or super acidic ion exchange resins such as nonporous or porous ion exchange resins each containing a strong acid group or a superacid group such as -$CF_3$, $CF_2$, or $SO_3H$; and heteropolyacids exemplified by polyacids containing an element such as P, Mo, V, W, or Si. Non-limiting examples of the strongly acidic or super acidic ion exchange resins include fluorinated sulfonic acid resins (fluorocarbon resins having a sulfo-containing group (a group containing a sulfonic group) in a side chain). Preferred, but non-limiting examples of the fluorinated sulfonic acid resins include copolymers between a sulfo-containing perfluorovinyl ether monomer and tetrafluoroethylene, such as Nafion® NR50 (supplied by Aldrich) and Nafion® H (supplied by E. I. du Pont de Nemours & Co.).

In particular, the support for use in the present invention is preferably selected from sulfated metal oxides, noble metals/sulfated metal oxides, metal oxide super strong acids, and strongly acidic or super acidic ion exchange resins (such as fluorinated sulfonic acid resins), and particularly preferably selected from sulfated metal oxides, noble metals/sulfated metal oxides, and fluorinated sulfonic acid resins. The sulfated metal oxides, noble metals/sulfated metal oxides, and fluorinated sulfonic acid resins have particularly high acid strengths and can thereby more effectively support the transition metal compound. Of the sulfated metal oxides, preferred examples include sulfated zirconia, sulfated tin, and sulfated hafnium. Of the noble metals/sulfated metal oxides, preferred examples include Pt/sulfated zirconia, Ir/sulfated zirconia, and Pd/sulfated zirconia. Among them, the support is advantageously selected from sulfated zirconia and fluorinated sulfonic acid resins, each of which is industrially easily available. In particular, the support is preferably selected from fluorinated sulfonic acid resins (such as Nation® and other copolymers between a sulfo-containing perfluorovinyl ether monomer and tetrafluoroethylene).

The support for use in the present invention is not limited in shape and particle size and can be selected from those having an appropriate shape and particle size suitable for the flow reactor. Non-limiting examples of the shape include shapes of pellets, powders, films, coatings, and any other shapes generally employed as solid catalysts.

The supporting of the transition metal in the form of an elementary substance, a compound, or an ion onto the support (such as an inorganic support, a sulfuric acid-supporting solid, or a solid super strong acid) may be performed by any of common processes such as impregnation, firing, precipitation, and ion exchange processes.

The transition metal compound may be supported in an amount of typically about 0.001 to about 20 weight percent, preferably about 0.01 to about 20 weight percent, and particularly about 0.1 to about 10 weight percent, in terms of the metal atom in the transition metal compound, relative to the weight of the support (such as an inorganic support, a sulfuric acid-supporting solid, or a solid super strong acid). When the support is ion-exchanged with the transition metal ion, the ion-exchange amount is typically about 0.001 to about 20 weight percent, preferably about 0.01 to about 20 weight percent, and particularly about 0.1 to about 10 weight percent, in terms of the metal atom in the transition metal ion, relative to the weight of the support (such as a sulfuric acid-supporting solid or a solid super strong acid).

The oxidation catalyst in the present invention is immobilized to or packed in the flow reactor. The immobilization or packing of the oxidation catalyst may be performed by a process publicly known or well known in the art. The oxidation catalyst may be packed into space in the flow reactor, or may be immobilized typically as a coating to the inner wall of the flow reactor.

In particular, in a preferred embodiment in the present invention, a coating is disposed on the inner wall of the flow reactor, where the coating includes a sulfuric acid-supporting solid or solid super strong acid being ion-exchanged with the transition metal ion. Especially, in a particularly preferred embodiment, a coating of a strongly acidic or super acidic ion exchange resin being ion-exchanged with the transition metal ion is disposed on the inner wall of the flow reactor. The flow reactor as mentioned above may be produced typically by coating the inner wall surface of a tube typically with a strongly acidic or super acidic ion exchange resin (such as a fluorinated sulfonic acid resin) and subjecting the resulting article to ion exchange with a transition metal compound solution (such as a cobalt compound solution). The tube may be made typically of a resin, glass, or a metal. The tube is preferably selected from tubes made of fluorocarbon resins such as polytetrafluoroethylenes (PTFEs).

Imide Compound Having Cyclic Imide Skeleton

An imide compound having a cyclic imide skeleton may be used herein as a promoter, so as to further promote the oxidation reaction. Specifically, the oxidation reactor may further include an imide compound inlet channel upstream from the flow reactor, where the imide compound having a cyclic imide skeleton is introduced from the imide compound inlet channel.

The imide compound having a cyclic imide skeleton can be selected from a variety of known imide compounds having a cyclic imide skeleton.

Of such imide compounds having a cyclic imide skeleton, representative, but non-limiting examples of compounds having a 5-membered cyclic imide skeleton include 5-membered cyclic N-hydroxyimide compounds such as N-hydroxysuccinimide, N-hydroxy-$\alpha$-methylsuccinimide, N-hydroxy-$\alpha,\alpha$-dimethylsuccinimide, N-hydroxy-$\alpha,\beta$-dimethylsuccinimide, N-hydroxy-$\alpha,\alpha,\beta,\beta$-tetramethylsuccinimide, N-hydroxymaleimide, N-hydroxyhexahydrophthalimide, N,N'-dihydroxycyclohexanetetracarboxylic diimide, N-hydroxyphthalimide, N-hydroxytetrabromophthalimide, N-hydroxytetrachlorophthalimide, HET acid N-hydroxyimide (N-hydroxy-1,4,5,6,7,7-hexachlorobicyclo[2.2.1]hept-5-ene-2,3-dicarboximide), himic acid N-hydroxyimide (N-hydroxy-bicyclo[2.2.1]hept-5-ene-2,3-dicarboximide, N-hydroxytrimellitimide, N,N'-dihydroxypyromellitic diimide, N,N'-dihydroxynaphthalenetetracarboxylic diimide, $\alpha,\beta$-diacetoxy-N-hydroxysuccinimide, N-hydroxy-$\alpha,\beta$-bis(propionyloxy)succinimide, N-hydroxy-$\alpha,\beta$-bis(valeryloxy)succinimide, N-hydroxy-$\alpha,\beta$-bis(lauroyloxy)succinimide, $\alpha,\beta$-bis(benzoyloxy)-N-hydroxysuccinimide, N-hydroxy-4-methoxycarbonylphthalimide, 4-ethoxycarbonyl-N-hydroxyphthalimide, N-hydroxy-4-pentyloxycarbonylphthalimide, 4-dodecyloxy-N-hydroxycarbonylphthalimide, N-hydroxy-4-phenoxycarbonylphthalimide, N-hydroxy-4,5-bis(methoxycarbonyl)phthalimide, 4,5-bis(ethoxycarbonyl)-N-hydroxyphthalimide, N-hydroxy-4,5-bis(pentyloxycarbonyl)phthalimide, 4,5-bis(dodecyloxycarbonyl)-N-hydroxyphthalimide, and N-hydroxy-4,5-bis(phenoxycarbonyl)phthalimide; and compounds corresponding to these compounds, except with the hydroxy group or groups bonded to the nitrogen atom being protected with a protecting group or groups.

Non-limiting examples of the protecting groups for the hydroxy groups include acyls such as acetyl, propionyl, and benzoyl; groups capable of forming an acetal or hemiacetal bond with the hydroxy group(s); sulfonyls such as methanesulfonyl and p-toluenesulfonyl; and groups resulting from removing OH group each from inorganic acids.

Of the imide compounds having a cyclic imide skeleton, representative, but non-limiting examples of compounds having a 6-membered cyclic imide skeleton include 6-membered cyclic N-hydroxyimide compounds such as N-hydroxyglutarimide, N-hydroxy-$\alpha,\alpha$-dimethylglutarimide, N-hydroxy-$\beta,\beta$-dimethylglutarimide, N-hydroxy-1,8-decahydronaphthalenedicarboximide, N,N'-dihydroxy-1,8;4,5-decahydronaphthalenetetracarboxylic diimide, N-hydroxy-1,8-naphthalenedicarboximide (N-hydroxynaphthalimide), and N,N'-dihydroxy-1,8;4,5-naphthalenetetracarboxylic diimide; and compounds corresponding to these compounds, except with the hydroxy group or groups bonded to the nitrogen atom being protected with a protecting group or groups. The protecting groups for the hydroxy groups are as described above.

Particularly preferred examples of the imide compounds having a cyclic imide skeleton include N-hydroxysuccinimide, N-hydroxyphthalimide, N,N'-dihydroxypyromellitic diimide, N-hydroxyglutarimide, N-hydroxy-1,8-naphthalenedicarboximide, and N,N'-dihydroxy-1,8;4,5-naphthalenetetracarboxylic diimide; and compounds resulting from introducing the protecting group or groups into the hydroxy group or groups of these N-hydroxyimide compounds.

Each of different imide compounds having a cyclic imide skeleton may be used alone or in combination.

The imide compound(s) having a cyclic imide skeleton may be used in an amount of typically about 0.0000001 to about 1 mole, preferably 0.00001 to 0.5 mole, and particularly preferably 0.0001 to 0.4 mole, per mole of the reaction substrate hydrocarbon. The imide compound(s) having a cyclic imide skeleton, when used in an amount within the range, allows the oxidation reaction to proceed at a high reaction rate.

The imide compound having a cyclic imide skeleton may be fed in the form of a solution in a solvent to the reaction system. The solvent for use herein may be selected from solvents inert to the reaction, such as the solvents listed in relation to the reaction substrate.

The oxidation reactor according to the present invention may further include a transition metal compound inlet channel upstream from the flow reactor. Through the transition metal compound inlet channel, a solution containing the transition metal compound is introduced so as to make up for the transition metal compound or the transition metal ion in the oxidation catalyst immobilized to or packed in the flow reactor. The solvent in the solution containing the transition metal compound may be selected from solvents inert to the reaction, such as the solvents listed in relation to the reaction substrate.

Oxidation Reaction

The reaction temperature in the oxidation reaction in the present invention can be selected as appropriate according to the types of the reaction substrate hydrocarbon and of the target product and is typically around room temperature to around 200°C, preferably 50°C to 130°C, and particularly preferably 60°C to 100°C. The reaction may be performed at normal atmospheric pressure or under pressure (under a load). When the reaction is performed under pressure (under a load), the reaction pressure is generally about 0.1 to about 10 MPa, preferably 0.15 to 8 MPa, and particularly preferably 0.5 to 8 MPa. The present invention allows the oxidation reaction to proceed smoothly even at normal atmospheric pressure (0.1 MPa), because of using the oxidizer oxygen in combination with ozone.

To the gas-liquid mixing unit 5, the gas is fed through the gas inlet channel 4, and the liquid is fed through the liquid inlet channel 15. The ratio of the flow rate (mL/min) of the gas to the flow rate (mL/min) of the liquid herein has only to be such a ratio that the gas-liquid slug flow is formed and the reaction efficiently proceeds. However, the ratio is generally from 2:8 to 8:2, preferably from 3:7 to 7:3, and more preferably from 4:6 to 6:4.

From the points typically of reaction efficiency and thermal efficiency, the gas-liquid slug flow in the flow reactor may flow at a linear velocity (LV) of typically 50 to 10000 cm/min, preferably 100 to 5000 cm/min, and more preferably 100 to 1000 cm/min.

The linear velocity (LV) (cm/min) of the gas-liquid slug flow may be determined according to the expression:

$$LV = (Q1 + Q2)/S$$

where:

Q1 represents the gas supply amount (the flow rate of the gas flowing through the gas inlet channel 4) ($cm^3$/min) ;
Q2 represents the liquid supply amount (the flow rate of the liquid flowing through the liquid inlet channel 15) ($cm^3$/min) ; and
S represents the cross-sectional area ($cm^2$) of the flow reactor.

The area S may be determined according to the expression:

$$S = (D/2)^2 \times \pi$$

where D represents the inside diameter (cm) of the flow reactor, where, when a coating is disposed on the inner wall, the "inside diameter" excludes the thickness of the coating.

The reaction mixture discharged from the flow reactor 7 is separated into a gas and a liquid in the gas-liquid separator 9, as described above. The separated liquid is recovered through the liquid recovery channel 16. When the one-pass conversion is low, at least part of the liquid may be recycled to the reaction system. In an embodiment, the reaction liquid may be recovered through the liquid recovery channel 16 after performing the operation of recycling the liquid separated in the gas-liquid separator 9 to the reaction system for a predetermined time. In another embodiment, a continuous system may be employed, in which part of the liquid separated in the gas-liquid separator 9 is continuously or intermittently drawn out (recovered) through the liquid recovery channel 16, while the remainder of the liquid is recycled to the reaction system.

The target oxide or oxides can be yielded by subjecting the recovered liquid to a separation process such as filtration, concentration, distillation, extraction, crystallization, recrystallization, or column chromatography, or a separation process as any combination of them.

Examples

The present invention will be illustrated in further detail with reference to several examples below. It should be noted, however, that the examples are by no means intended to limit the scope of the present invention.

Example 1

Tetradecane was oxidized according to the flow illustrated in Fig. 1. Initially, the oxidation reactor will be described. Oxygen is fed from an oxygen cylinder (not shown) through the oxygen inlet channel 2 to the ozone generator 3 and partially converted into ozone. Into this, nitrogen is introduced from a nitrogen supply line (not shown) to form a mixed gas containing oxygen, ozone, and nitrogen (oxygen-ozone-nitrogen mixed gas). The mixed gas is fed through the gas inlet channel 4 to the gas-liquid mixer (gas-liquid mixing unit) 5, which is made of Daiflon. Independently, the reaction substrate tetradecane (liquid) is fed through the hydrocarbon supply channel 1 to the gas-liquid mixer 5. However, the hydrocarbon supply channel 1 was not used in this example. Fig. 2 is a schematic view of the gas-liquid mixer 5 when viewed laterally. The upside of Fig. 2 corresponds to the upside of the gas-liquid mixer. Fig. 3 is a schematic view of the gas-liquid mixer 5 when viewed from above. The gas-liquid mixer 5 includes a cylindrical channel (gas-liquid mixing zone) 51 disposed so that the axis of the cylinder lies horizontally. In the cylindrical channel 51, fluids flow in the axial direction of the cylinder (namely, flow horizontally). The cylindrical channel 51 has an inside diameter of 2.0 mm and a length of 3.0 mm. The cylindrical channel 51 internally houses one glass spherical particle 52. The particle 52 has a diameter of 1.2 mm and is disposed rotatably and/or movably. The inlet channel 53 is disposed at one end in the lengthwise direction of the cylindrical channel 51; and the outlet channel 54 is disposed at the other end. The inlet channel 53 has a circular cross-section and an inside diameter of 1.0 mm. The outlet channel 54 has a circular cross-section and an inside diameter of 1.0 mm. To the inlet channel 53, the gas inlet channel 4 and the after-mentioned liquid inlet channel 15 are coupled. The outlet channel 54 is coupled to the after-mentioned gas-liquid mixture channel 6.

The oxygen-ozone-nitrogen mixed gas and the tetradecane fed to the cylindrical channel 51 of the gas-liquid mixer 5 are mixed with each other via gas-liquid mixing by the rotation and/or movement of the spherical particle 52 within the cylindrical channel 51, to form a gas-liquid slug flow (gas-liquid alternate flow), and the gas-liquid slug flow is discharged through the outlet channel 54 and forwarded through the gas-liquid mixture channel 6 to the flow reactor 7.

The flow reactor 7 was prepared in the following manner. Specifically, a 20 weight percent Nafion dispersion (supplied by E. I. du Pont de Nemours & Co.) was allowed to flow through a Teflon® tube having an inside diameter of 2.0 mm and a length of 1.5 m, the resulting article was dried at about 100°C, and yielded a Teflon® tube bearing a 0.5-mm thick Nafion coating on its inner wall. The resulting tube had an inside diameter of 1.0 mm. Through this tube, a 5 weight percent solution of cobalt acetate ($Co(OAc)_2$) in acetic acid was allowed to flow, the resulting article was heated at about 100°C for one hour, dried at 120°C, and yielded a flow reactor bearing, on the inner wall, a coating of a fluorinated sulfonic acid resin being ion-exchanged with cobalt ions. The fluorinated sulfonic acid resin coating has a cobalt concentration of 2.4 weight percent. All (100%) of ion exchange groups of Nafion are replaced (exchanged) with cobalt ions.

Part of the flow reactor 7 corresponding to a length of 1 m is immersed in an oil bath (at 90°C) equipped with a temperature controller. The reaction mixture discharged from the flow reactor 7 is introduced from the reaction mixture channel 8 to the gas-liquid separator 9 and is separated into a gas and a liquid, where the gas-liquid separator 9 is a steel-use stainless (SUS) vessel having an inner capacity of 100 mL. The separated gas is introduced into the ozone decomposer 12 and discharged through the exhaust gas line 13 to the outside. Part of the separated gas is recycled through the gas circulation channel 11 to the reaction system as needed. However, the gas was not recycled in this example. The liquid separated in the gas-liquid separator 9 can be recycled through the liquid circulation channel 10 and the liquid inlet channel 15 to the gas-liquid mixer 5, using a pump for high-performance liquid chromatographs (not shown). The liquid separated in the gas-liquid separator 9 can also be recovered through the liquid recovery channel 16. As needed, an imide compound having a cyclic imide skeleton may be fed through the imide compound inlet channel 14 and the liquid inlet channel 15 to the gas-liquid mixer 5. However, the imide compound was not used in this example.

Using the above-mentioned oxidation reactor, the oxidation reaction of tetradecane was performed. Specifically, 20 g of tetradecane were placed in the gas-liquid separator 9 and heated up to 90°C with stirring in a nitrogen atmosphere. Oxygen was fed through the oxygen inlet channel 2 to the ozone generator 3, where part of which oxygen was converted into ozone, and then mixed with nitrogen to give a oxygen-ozone-nitrogen mixed gas containing 20.5 volume percent of oxygen, 0.5 volume percent of ozone, and 79.0 volume percent of nitrogen. The mixed gas was fed through the gas inlet channel 4 to the gas-liquid mixer 5 at a flow rate of 10 mL/min. The tetradecane in the gas-liquid separator 9 was fed through the liquid circulation channel 10 and the liquid inlet channel 15 to the gas-liquid mixer 5 at a flow rate of 10 mL/min. The mixed gas and tetradecane fed to the gas-liquid mixer 5 were mixed with each other via gas-liquid mixing by the rotation and/or movement of the mobile particle 52 within the cylindrical channel 51 and formed a gas-liquid slug flow. The gas-liquid slug flow was fed from the outlet channel 54 through the gas-liquid mixture channel 6 to the flow reactor 7. The reaction mixture discharged from the flow reactor 7 was fed through the reaction mixture channel 8 to the gas-liquid separator 9 and separated into a gas and a liquid. The gas separated in the gas-liquid separator 9 was introduced into the ozone decomposer 12 and discharged through the exhaust gas line 13 to the outside. The liquid separated in the gas-liquid separator 9 was recycled through the liquid circulation channel 10 and the liquid inlet channel 15 to the gas-liquid mixer 5, using the pump. After performing this operation for 8 hours, the pump was stopped, and the composition (formulation) of a liquid in the gas-liquid separator 9 was analyzed by gas chromatography (column: DB-1, supplied by Agilent Technologies) and was found that the conversion from tetradecane was 2.9%; and that tetradecanol, tetradecanone, and tetradecanoic acid were formed as oxides (oxidation products) of tetradecane.

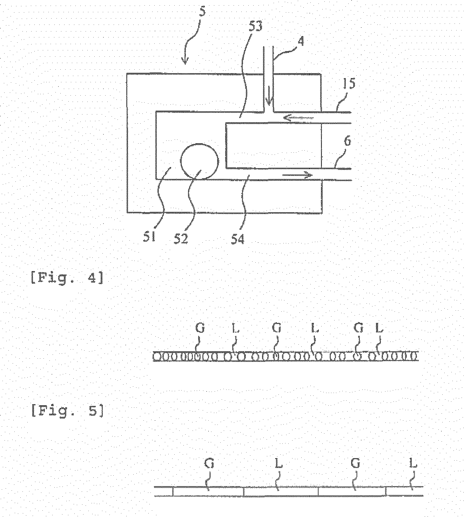

A model experiment was also performed by the above-mentioned operation, except for using a transparent Teflon® tube (supplied by Flonchemical Co., Ltd., having an inside diameter of 1.0 mm) instead of the flow reactor 7, using air instead of the oxygen-ozone-nitrogen mixed gas, feeding the air at a flow rate of 10 mL/min, and feeding tetradecane at a flow rate of 10 mL/min. In this experiment, a fluid flowing through the transparent tube was visually observed to find that an extremely fine gas-liquid slug flow (gas-liquid alternate flow) as illustrated in Fig. 4 was formed. Gas portions had lengths (diameters of bubbles) in the channel direction of 1 mm or less.

Comparative Example 1

Tetradecane oxidation was performed by a procedure as in Example 1, except for using a commercially available T-mixer (made of Teflon®, supplied by Flonchemical Co., Ltd., having an inside diameter of 1.0 mm) instead of the gas-liquid mixer 5. The T-mixer was configured so that the mixed gas was fed from the lower part of "T", tetradecane was fed from the left hand of "T", the two components were merged at the crossing of "T", and the merged fluid was allowed to flow toward the right hand of "T".

As a result, it was found that the conversion from tetradecane was 2.0%; and that tetradecanol, tetradecanone, and tetradecanoic acid were formed as oxidation products.

A model experiment was also performed by the above-mentioned operation, except for using a transparent Teflon® tube (supplied by Flonchemical Co., Ltd., having an inside diameter of 1.0 mm) instead of the flow reactor 7, using air instead of the oxygen-ozone-nitrogen mixed gas, feeding the air at a flow rate of 10 mL/min, and feeding tetradecane at a flow rate of 10 mL/min. In the experiment, a fluid flowing through the transparent tube was visually observed and was found that a gas-liquid slug flow (gas-liquid alternate flow) as illustrated in Fig. 5 was formed. Gas portions and liquid portions were found to have lengths in the channel direction each of about 9 mm on average.

Example 2

An operation as in Example 1 was performed, except for using 2,2,4-trimethylpentane as a reaction substrate instead of tetradecane. As a result, the substrate conversion was found to be 3.8%.

Example 3

An operation as in Example 1 was performed, except for using n-heptane as a reaction substrate instead of tetradecane. As a result, the substrate conversion was found to be 2.9%.

Example 4

An operation as in Example 1 was performed, except for using toluene as a reaction substrate instead of tetradecane. As a result, the substrate conversion was found to be 19.3%.

Example 5

An operation as in Example 1 was performed, except for using 2-methylbiphenyl as a reaction substrate instead of tetradecane. As a result, the substrate conversion was found to be 19.3%.

Industrial Applicability

According to the present invention, a mobile particle is disposed in a channel (gas-liquid mixing zone) of the gas-liquid mixing unit and rotates and/or moves to form a fine gas-liquid slug flow (or gas-liquid alternate flow), and this gas-liquid slug flow is introduced into the flow reactor bearing or including the oxidation catalyst. Further, both oxygen and ozone are used as oxidizers. Thus, reaction components can move quickly, and the oxidation can proceed rapidly at a high rate. Accordingly, with the present invention, a wide variety of hydrocarbons, in particular even straight chain alkanes, can be efficiently oxidized. In addition, since heat exchange can be smoothly performed, the reaction heat can be efficiently removed, and high energy efficiency is offered. In addition and advantageously, the oxidation apparatus can be downsized.

Reference Signs List

| | |
|---|---|
| 1 | hydrocarbon supply channel |
| 2 | oxygen inlet channel |
| 3 | ozone generator |
| 4 | gas inlet channel |
| 5 | gas-liquid mixing unit (gas-liquid mixer) |
| 6 | gas-liquid mixture channel |
| 7 | flow reactor |
| 8 | reaction mixture channel |
| 9 | gas-liquid separator |
| 10 | liquid circulation channel |
| 11 | gas circulation channel |
| 12 | ozone decomposer |
| 13 | exhaust gas line |
| 14 | imide compound inlet channel |
| 15 | liquid inlet channel |
| 16 | liquid recovery channel |
| 51 | channel in gas-liquid mixing unit (gas-liquid mixer) |
| 52 | mobile particle |
| 53 | inlet channel |
| 54 | outlet channel |
| G | gas portion |
| L | liquid portion |

**Claims**

1. An oxidation reactor comprising:

a liquid inlet channel through which a liquid containing a reaction substrate hydrocarbon is introduced;
a gas inlet channel through which a gas containing oxygen and ozone is introduced;
a gas-liquid mixing unit that mixes the liquid introduced from the liquid inlet channel with the gas introduced from the gas inlet channel;
a flow reactor to or in which an oxidation catalyst is immobilized or packed; and
a mobile particle disposed in a channel of the gas-liquid mixing unit, the mobile particle capable of rotating and/or moving to mix the liquid with the gas to thereby form a gas-liquid slug flow, the gas-liquid slug flow being introduced into the flow reactor.

2. The oxidation reactor according to claim 1, further comprising
a gas-liquid separator downstream from the flow reactor.

3. The oxidation reactor according to claim 2, further comprising
a circulation channel through which at least part of a liquid separated in the gas-liquid separator is recycled to the gas-liquid mixing unit or to an upstream portion from the gas-liquid separator.

4. The oxidation reactor according to one of claims 2 and 3, further comprising
a circulation channel through which at least part of a gas separated in the gas-liquid separator is recycled to the gas-liquid mixing unit or to an upstream portion from the gas-liquid mixing unit.

5. The oxidation reactor according to any one of claims 1 to 4,
wherein the oxidation catalyst is a catalyst comprising
a transition metal in an form of an elementary substance, a compound, or an ion; and
an inorganic support onto which the transition metal is supported or immobilized.

6. The oxidation reactor according to any one of claims 1 to 4,
wherein the oxidation catalyst is selected from:

a catalyst comprising:

a transition metal compound; and
a support having a Hammett acidity function $(H_0)$ of -9 or less and supporting the transition metal compound; and

a catalyst comprising:

a transition metal ion; and
a support having a Hammett acidity function $(H_0)$ of -9 or less and being ion-exchanged with the transition metal ion.

7. The oxidation reactor according to claim 6,
wherein the support comprises a strongly acidic or super acidic ion exchange resin.

8. The oxidation reactor according to any one of claims 1 to 4, 6, and 7,
wherein the flow reactor comprises
a coating disposed on an inner wall of the flow reactor, the coating comprising:

a transition metal ion; and
a strongly acidic or super acidic ion exchange resin being ion-exchanged with the transition metal ion.

9. The oxidation reactor according to any one of claims 1 to 8, further comprising
an imide compound inlet channel upstream from the flow reactor, where an imide compound having a cyclic imide skeleton is introduced from the imide compound inlet channel.

10. A method for producing an oxide, the method comprising oxidizing a hydrocarbon in coexistence of oxygen and ozone using the oxidation reactor according to any one of claims 1 to 9, to yield a corresponding oxide or oxides.

[Fig. 1]

[Fig. 2]

[Fig. 3]

[Fig. 4]

[Fig. 5]

## INTERNATIONAL SEARCH REPORT

| International application No. |
| --- |
| PCT/JP2015/072503 |

A. CLASSIFICATION OF SUBJECT MATTER

*C07C27/16*(2006.01)i, *C07C29/48*(2006.01)i, *C07C31/125*(2006.01)i, *C07C45/40*
(2006.01)i, *C07C49/04*(2006.01)i, *C07C51/34*(2006.01)i, *C07C53/126*(2006.01)i,
*B01F3/04*(2006.01)n, *B01F13/00*(2006.01)n, *B01J32/00*(2006.01)n,

According to International Patent Classification (IPC) or to both national classification and IPC

B. FIELDS SEARCHED

Minimum documentation searched (classification system followed by classification symbols)
C07C27/00-71/00, B01F3/00-3/22, 13/00-13/10, B01J32/00, C07B61/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2006-257001 A (Mitsubishi Chemical Corp.), 28 September 2006 (28.09.2006), claims; paragraphs [0031], [0037], [0047] to [0049]; examples; drawings (Family: none) | 1-10 |
| A | JP 2008-280328 A (Sumitomo Chemical Co., Ltd.), 20 November 2008 (20.11.2008), claims; paragraphs [0009], [0013], [0016]; examples & CN 101284760 A & EP 1980549 A1 & KR 10-2008-0092855 A & TW 200902487 A & US 2008/0255392 A1 claims; paragraphs [0010], [0014], [0017]; examples | 1-10 |

☒ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 October 2015 (15.10.15) | 27 October 2015 (27.10.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

**INTERNATIONAL SEARCH REPORT**

International application No.

PCT/JP2015/072503

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2008-201723 A  (Fuji Xerox Co., Ltd.), 04 September 2008 (04.09.2008), claims; paragraphs [0030] to [0032]; examples; drawings (Family: none) | 1-10 |
| A | WO 2009/048141 A1  (Nippon Soda Co., Ltd. et al.), 16 April 2009 (16.04.2009), claims; paragraph [0018]; examples; drawings & CN 101820995 A          & EP 2206550 A1 & JP 5598952 B2          & KR 10-2010-0053679 A & US 2010/0210876 A1 claims; paragraph [0032]; examples; drawings | 1-10 |

Form PCT/ISA/210 (continuation of second sheet) (July 2009)

**INTERNATIONAL SEARCH REPORT**

International application No.

PCT/JP2015/072503

Continuation of A. CLASSIFICATION OF SUBJECT MATTER
 (International Patent Classification (IPC))

*C07B61/00*(2006.01)n

            (According to International Patent Classification (IPC) or to both national
            classification and IPC)

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014175313 A

- JP 2002331242 A